# EUROPÄISCHE PATENTSCHRIFT

(11) **EP 1 243 644 B1**
(45) Veröffentlichungstag und Bekanntmachung des Hinweises auf die Patenterteilung: **31.05.2006**
(21) Anmeldenummer: 01106958.0
(22) Anmeldetag: 20.03.2001
(51) Int. Cl.: C12C 11/00, G01N 21/90, G01N 33/14

(54) **Verfahren zur Untersuchung von Zuständen in einem Behältnis und Vorrichtungen zur Durchführung des Verfahrens**
Method for investigating the conditions in a container and devices for carrying out the method
Procédé pour examiner les conditions dans un récipient et systemes pour la réalisation de ce procédé

(43) Veröffentlichungstag der Anmeldung: 25.09.2002
(73) Patentinhaber: Syskron GmbH, 93068 Neutraubling (DE)
(72) Erfinder: Fahrner, Hartmut, 84028 Landshut (DE)
(74) Vertreter: Grünecker, Kinkeldey, Stockmair & Schwanhäusser Anwaltssozietät

(56) Entgegenhaltungen:
- DE-A- 19 828 688
- DE-A- 19 842 989
- US-A- 4 959 537

## Beschreibung

Die Erfindung betrifft ein Verfahren zur Untersuchung mindestens eines Zustands in einem Behältnis, wie es bei der Getränkeherstellung bei der Vermehrung und/oder Vergärung von/mit Organismen verwendet wird, wie z.B. einem Gärtank, Hefereinzuchttank, biologischen Milchsäuretank, Lagertank oder dergleichen. Das Verfahren kann insbesondere bei der Herstellung von Bier durchgeführt werden.

Bekannt ist nach der DE 198 28 688 ein Verfahren zur Steuerung des Gärprozesses bei der Getränkeherstellung, bei dem optisch aufgenommene Signale erzeugt werden und zur Steuerung der Gärprozesse in einer Vielzahl von Gärgefäßen herangezogen werden.

Bei diesem Verfahren wird ein Wechsel der Farbe und Struktur der Oberfläche der gärenden Würze optisch aufgenommen und mit bekannten Mustern bzw. Farbhistogrammen verglichen.

Aus der DE 198 42 989 A1 ist eine Brauereinanlage mit Kameraüberwachung bekannt. Aus des US 4 959 537 ist ein Verfahren und eine Vorrichtung zur Inspektion von durchsichtigen Behälten bekannt. Aufgabe der vorliegenden Erfindung ist es, ein Verfahren zu schaffen, mit dem eine große Anzahl von möglichen Zuständen in einem Behältnis, wie es bei der Getränkeherstellung verwendet wird, erkannt werden können und das mit hoher Genauigkeit und guter Reproduzierbarkeit.

Die Zustände, die erkannt werden sollen, sind
- der Zustand des Schauglases, und/oder
- Nebel im Behältnis, und/oder
- der Reinigungszustand des Behältnisses, und/oder
- Befüllung, und/oder
- Höhe der Oberfläche eines Mediums innerhalb des Behältnisses, und/oder
- der Zustand der Belüftung, und/oder
- das Eintauchen einer Sprühkugel in ein Medium, das sich innerhalb des Behältnisses befindet, und/oder
- das Leersein des Behältnisses, und/oder
- der Zustand der Fermentation, wie
   - das Ende der Gärung, und/oder
   - das Überweissen und/oder
   - der Zustand der Hauptgärung.

Diese Aufgabe wird durch ein Verfahren mit den Merkmalen des Anspruchs 1 und durch eine Vorrichtung mit den Merkmalen des Anspruchs 38 gelöst.

Bei dem erfindungsgemäßen Verfahren werden zur Untersuchung des Zustandes in einem Behältnis, wie es bei der Geträmkeherstellung verwendet wird, die folgenden Schritte durchgeführt:
Zunächst wird ein Bild, das aus Pixeln zusammengesetzt ist, erstellt. Das Erstellen des Bildes kann z.B. das Aufnehmen eines Bildes mit einer Kamera sein oder auch das Errechnen eines Bildes aus einem aufgenommenen Bild durch bildverarbeitende Verfahren wie z.B. Subtraktion eines Referenzbildes, Scharfzeichnen, Weichzeichnen, Kontrastveränderung, etc..

Anschließend werden ein oder mehrere Bereiche des Bildes durch Auswahl von Pixeln bestimmt. Das Bestimmen eines Bereiches erfolgt durch Auswählen von einer Gruppe von Pixeln des Bildes. Die Gruppe von Pixeln kann zusammenhängend oder auch separiert sein. Die Auswahl erfolgt in der Regel durch Angabe von Koordinaten, die einen zu bestimmenden Bereich charakterisieren. Dies können z.B. die Endpunkte einer Linie, die Eckpunkte eines Rechtecks oder der Radius und Mittelpunkt eines Kreises sein.

Weiterhin wird ein Kriterium vorgegeben, anhand dessen die Pixel in den bestimmten Bereichen analysiert werden. Das Analysieren beschreibt das Prüfen ob ein Pixel ein vorgegebenes Kriterium erfüllt oder nicht oder auch wie stark ein Kriterium erfüllt wird. Dazu können die Pixel auch gruppenweise zusammengefasst werden und zusammengenommen auf die Erfüllung eines Kriteriums hin überprüft werden.
Das Kriterium kann spezielle Farbwerte oder die Ableitung bestimmter Farbwerte entlang einer spezifizierten Richtung betreffen. So ist ein mögliches Kriterium beispielsweise, dass die Helligkeit eines Pixels einen Mindestwert überschreitet.

Als nächstes wird ein Maß festgestellt, das sich aus der Lage der Pixel oder aus der Anzahl bzw. der relativen Anzahl der Pixel innerhalb des bestimmten Bereiches, die das Kriterium erfüllen, ergibt.
Die Lage der Pixel ist beispielsweise durch ihre Lage relativ zu einem vorgegebenen Pixel charakteristisch für einen Zustand in dem Behältnis. Auch die Lage von Pixeln, die das Kriterium erfüllen, relativ zueinander kann charakteristisch für einen Zustand in dem Behältnis sein.
Ist das Kriterium z.B. die Farbigkeit, so kann das Feststellen des Maßes auch dadurch erfolgen, das festgestellt wird, wie farbig Pixel sind. Dies erfolgt beispielsweise durch Feststellen der Farbigkeit als Prozentzahl des Maximalwerts auf einer Farbigkeitsskala.

Die Anzahl der Pixel, die ein Kriterium erfüllen, sind ebenfalls charakteristisch für Zustände in einem Behältnis. Wenn beispielsweise viele Pixel das Kriterium einer Mindesthelligkeit erfüllen, so kann ein Maß festgestellt werden, das die Anzahl der Pixel angibt, die innerhalb des bestimmten Bereichs liegen und die Mindesthelligkeit aufweisen.

Aus dem festgestellten Maß wird schließlich auf den Zustand in dem Behältnis geschlossen. Dieser Schluss kann beispielsweise der Schluss auf das Einsetzen der Gärung oder auf das Ende der Gärung sein. Der Schluss kann auch das Schließen auf eine Wahrscheinlichkeit sein, mit der ein Zustand (z.B. "Ende der Gärung") vorliegt. Auch ist es erfindungsgemäß möglich, den Schluss zu ziehen, dass die Oberfläche eines Mediums in dem Behältnis eine gewisse Höhe erreicht hat.

Die Vorteile dieses Verfahrens liegen darin, dass bestimmte Bereiche des Bildes, die besonders geeignet sind, den Zustand in dem Behältnis zu qualifizieren, herausgehoben werden können und speziell analysiert werden können.
Weiter ist es durch Vorgabe eines oder mehrerer Kriterien möglich, verschiedenartigste Zustände in dem Behältnis sicher zu untersuchen.

Hierbei ist es vorteilhaft, die Lage der Pixel, die das vorgegebene Kriterium erfüllen, zu analysieren und auf Grundlage der Lage der Pixel ein Maß festzustellen. Anhand des festgestellten Maßes kann auf den Zustand in dem Behältnis geschlossen werden. Vorteilhafterweise wird mit Hilfe der Anzahl der Pixel, die ein Kriterium erfüllen auf einen Zustand geschlossen werden. Dies erlaubt sehr schnelle Bearbeitungszeiten und ermöglicht eine schnelle Anpassbarkeit an verschiedene Betriebsbedingungen bei verschiedenen Verfahren in einem Behältnis, wie es in bei der Getränkeherstellung verwendet wird.
Durch Festlegen verschiedener Kriterien und verschiedener Möglichkeiten aus dem festgestellten Maß auf den Zustand in dem Behältnis zu schließen, kann das erfindungsgemäße Verfahren für vielfältige verschiedenartige Vorgänge in einem Behältnis, insbesondere bei der Gärung von Bier, die bei verschiedenen Biersorten vorkommen können, angepasst werden.

Die folgenden Ausführungen beziehen sich der Klarheit halber auf einen Gärtank, jedoch ist darunter immer ein wie Eingangs beschriebenes Behältnis, wie es bei der Getränkeherstellung bei der Vermehrung und/oder Vergärung von/mit Organismen verwendet wird, wie z.B. ein Gärtank, Hefereinzuchttank, biologischer Milchsäuretank, Lagertank oder dergleichen zu verstehen.

Vorteilhafterweise betrifft das Kriterium, anhand dessen die Pixel in dem bestimmten Bereich analysiert werden, den R-, G- und/oder B-Farbwert (R = Rot, G = Grün, B = Blau) im RGB-Farbsystem und/oder den H-, S- und/oder I-Farbwert im HSI (Hue, Saturation, Intensity) -Farbsystem. Durch die verschiedenen Farbwerte können verschiedene Eigenschaften der dargestellten Objekte ausgenutzt werden, um den Zustand in einem Gärtank erfindungsgemäß zu untersuchen. Eine Farbinformation im RGB-Farbsystem kann in eine Farbinformation im HSI-Farbsystem umgerechnet werden und umgekehrt.

Ein weiteres Kriterium, das vorteilhafterweise herangezogen werden kann, ist die Farbigkeit. Diese ist definiert als die Summe der Absolutwerte der Differenzen zwischen dem R-, G- und B-Farbwert.
Die Farbwerte können beispielsweise in Skalen von 0 bis 255 angegeben sein, wobei 255 ein Maximalwert des Farbwerts entspricht.
Vorteilhafterweise kann weiterhin ein Kriterium die Übersteuerung von Pixeln betreffen. Die Übersteuerung von Pixeln ist dadurch gegeben, dass sowohl R- als auch G- und B-Farbwert einen vorgegebenen Wert überschreiten, wobei dies beispielsweise 90 % des Maximalwerts sein kann.

Die einfachste Weise, ein Bild zu erstellen, besteht darin, das Bild aufzunehmen; jedoch ist es vorteilhafterweise auch möglich, das aufgenommene Bild zunächst weiterzuverarbeiten, indem z.B. von dem aufgenommenen Bild ein Referenzbild insbesondere pixelweise abgezogen wird. Hierbei wird von einem aufgenommenen Bild von jedem R-, G- und B-Farbwert eines Pixels der R-, G- und B-Farbwert eines Pixels eines Referenzbildes abgezogen und so das Bild erstellt. Auch ist es vorteilhafterweise möglich, das Bild dadurch zu erstellen, dass ein aufgenommenes Bild im Kontrast verstärkt oder emiedrigt, in der Helligkeit verstärkt oder emiedrigt und in Bezug auf Schärfe bearbeitet wird.

Dadurch lassen sich störende Bildbestandteile, die eine weitere Bearbeitung oder Analyse des Bildes erschweren würden, aufheben.

Vorteilhafterweise wird das Bild von oben in Richtung des unteren Teiles des Tanks aufgenommen. So kann sowohl die Innenseite des Gärtanks als auch die Oberfläche des Mediums, das sich im Gärtank befindet, aufgenommen werden. Weiterhin kann so auch ein Bild der Sprühkugel aufgenommen werden. Die Analyse jedes einzelnen Bildbereiches ist vorteilhaft für die Untersuchung der verschiedenen Zustände in dem Gärtank.

Die bevorzugten Bildbereiche, die in dem erfindungsgemäßen Verfahren bestimmt werden, betreffen das Abbild des Konuses, des Sprühkopfes und des Brandthefebereiches. Der Konus ist ein am unteren Teil des Gärtanks vorgesehener konusförmiger Bereich, der in den aufgenommenen Bildern in der Regel etwa in der Mitte des Bildes wiederzufinden ist. Der Sprühkopf ist eine im oberen Bereich des Gärtanks angebrachte Vorrichtung, mit der beispielsweise Reinigungsmittel in den Gärtank eingebracht werden kann. Der Brandthefebereich ist derjenige Bereich, bei dem während des Gärprozesses Schaum Kontakt mit dem Gärtank hat. Insbesondere in diesem Bereich bleibt nach der Gärung ein verschmutzter Bereich (der Brandthefebereich) zurück.

Vorteilhafterweise kann der erfindungsgemäße Bildbereich einen oder mehrere einzelne Teilbildbereiche umfassen.

Die bestimmten Bildbereiche oder die Teilbildbereiche können sich durch Auswählen oder Weglassen eines kreisförmig, linienförmig, quadratisch, rechteckig, n-eckig oder unregelmäßig geformten Bildbereiches ergeben. Linien können hierbei beispielsweise nur eine Breite von 1 Pixel haben, jedoch kann eine Linie auch eine Breite von mehreren Pixel haben. Dabei ist es dann vorteilhaft die Pixelwerte senkrecht zur Linienrichtung aufzuaddieren.

Für Untersuchungen des Bildbereiches des Konuses ist es beispielsweise vorteilhaft, für einen zylindrischen Gärtank einen kreisförmigen Bildbereich zu bestimmen.

Für die Untersuchung des Brandthefebereiches ist es vorteilhaft, einen kreisförmigen Bildbereich wegzulassen, und zur Untersuchung von beispielsweise Kontraständerungen ist es vorteilhaft, linienförmige Bildbereiche zu bestimmen.

Vorteilhafterweise kann mit dem Verfahren der Zustand des Schauglases, von Nebel im Gärtank, der Sauberkeit nach der Reinigung, der Befüllung, der Höhe der Oberfläche eines Mediums innerhalb des Gärtanks, der Belüftung, des Eintauchens einer Sprühkugel in ein Medium innerhalb des Gärtanks, des Leerseins des Gärtanks, der Fermentation wie des Endes der Gärung, des Überweissens und/oder der Hauptgärung untersucht werden. Jede dieser Zustände ist ein Zustand, der für die Gärung, insbesondere bei der Herstellung für Bier, von größtem Interesse ist.

Eine vorteilhafte Ausführungsform des erfindungsgemäßen Verfahrens besteht darin, dass der Schluss auf den Zustand in dem Gärtank dadurch erfolgt, dass das Ermitteln des Vorliegens eines Zustandes geschieht. Eine weitere vorteilhafte Ausführungsform besteht darin, eine Wahrscheinlichkeit für das Vorliegen eines Zustandes zu schließen. Mit dem Schließen einer Wahrscheinlichkeit können andere mit dem erfindungsgemäßen Untersuchungsverfahren gewonnenen Ergebnisse hinzugezogen werden, um auf eine geänderte Wahrscheinlichkeit zu schließen oder auf das definitive Vorliegen eines Zustandes zu schließen.

Hierbei sind auch die Einbeziehungen von Parametern, die anderweitig gewonnen werden (z.B. Druck oder Temperatur im Gärtank), möglich.

Vorteilhafterweise ist das Maß, welches festgestellt wird, einer Prozentzahl gleichzusetzen, so dass dieses Maß beispielsweise zwischen 0 und 1 bzw. zwischen 0 und 100 liegt. Dementsprechend kann das Schließen auf eine Wahrscheinlichkeit für das Vorliegen eines Zustandes durch Gleichsetzen der Wahrscheinlichkeit mit dem festgestellten Maß erfolgen.

Vorteilhafterweise kann die Untersuchung auf den Zustand in einem Gärtank mit einem oder mehreren Bildern erfolgen. Bei der Verwendung von mehreren Bildern ist insbesondere der zeitliche Abstand zu berücksichtigen.

Eine weitere vorteilhafte Ausführung des erfindungsgemäßen vertanrens besteht dann, die Koordinaten und/oder den Radius von Konus und/oder einer Sprühkugel vorzugeben. Dadurch ist die Bestimmung von Bereichen des Bildes einfach und schnell möglich.

Eine vorteilhafte Ausführungsform besteht auch darin, dass Kontrast und Helligkeit des Bildes, das aufgenommen wird, vorgegeben sind, so dass sich auch für verändernde Beleuchtungsverhältnisse vergleichbar auswertbare Bilder ergeben.

Eine besonders vorteilhafte Ausführungsform des Verfahrens besteht darin, den Zustand des Schauglases zu untersuchen, indem ein Bildbereich bestimmt wird, in dem bei einem sauberen und durchsichtigen Schauglas ein hoher Intensitätsunterschied auftritt. Dies ist besonders im Bereich des Abbilds des Sprühkopfes und einer Zuleitung zu dem Sprühkopf möglich. Vorteilhafterweise werden hierbei Bereiche bestimmt, die die Form von Linien haben und das Kriterium betrifft, den Intensitätsunterschied zwischen den Intensitäten der Pixel der Linien eines Linienpaares. Weiterhin ist es vorteilhaft zu analysieren, ob der Intensitätsunterschied bestimmter Pixel einen vorbestimmten Wert über- bzw. unterschreiten und das Kriterium als erfüllt anzusehen, falls sich nicht bereits bei einem anderen Pixel derselben Linie ergeben hat, dass der vorbestimmten Wert über- bzw. unterschritten ist. Da bei einem verschmutzten Schauglas normalerweise ein unscharfes Bild aufgenommen wird (das Schauglas ist außerhalb des Fokusbereichs der Kamera), sind die Helligkeitsunterschiede des aufgenommenen Bildes stark verwaschen, so dass die Bestimmung eines starken Helligkeitsunterschiedes innerhalb des Bildes darauf hindeutet, dass das Schauglas nicht beschmutzt sondern durchsichtig ist.

Eine weitere vorteilhafte Ausführungsform der Erfindung besteht darin, dass das Kriterium die Ableitung der Intensität entlang der Linienrichtung für eine vorbestimmte Anzahl von Linien betrifft und dass das Kriterium für ein Pixel erfüllt ist, falls der Absolutwert der Ableitung der Intensität bei diesem Pixel für die Pixel einer Linie maximal ist und einen vorbestimmten Wert überschreitet.
Aus derartigen Informationen kann insbesondere auf das Vorhandensein von Nebel in dem Gärtank geschlossen werden.

Eine weitere vorzugsweise Ausführungsform des Verfahrens besteht darin, dass das Kriterium darin besteht, dass die Farbe der Pixel in dem bestimmten Bereich einen gelblich-braunen Farbton haben, wie er für Brandthefe üblicherweise gefunden wird. Dadurch kann vorteilhafterweise der Teil der Innenseite des Gärtanks, der mit Brandthefe belegt ist, quantitativ bestimmt werden, da die Innenseite eines sauberen Stahltanks einen eher metallisch-grauen Farbton aufweist.

Eine weitere besonders vorteilhafte Ausführungsform des Verfahrens besteht darin, mehrere Maße festzustellen und aufgrund von mehreren festgestellten Maßen auf den Zustand in dem Gärtank zu schließen. Durch das Heranziehen von mehreren festgestellten Maßen ist es möglich, eine genauere und zuverlässigere Aussage über den Zustand in dem Gärtank zu gewinnen, als nur durch Heranziehen eines festgestellten Maßes.

Eine weitere vorteilhafte Ausführungsform des Verfahrens besteht darin, dass auf die Wahrscheinlichkeit für das Überweissen dadurch geschlossen wird, dass auf die Wahrscheinlichkeit für das Ende der Gärung geschlossen wird und auf die Wahrscheinlichkeit für Überweissen gleich 1 minus der Wahrscheinlichkeit für das Ende Gärung geschlossen wird.

Eine weitere vorteilhafte Ausführungsform der Erfindung besteht darin, dass auf den Radius des Abbildes der Oberfläche des Füllmediums des Gärtanks und vorzugsweise damit auf die Höhe der Oberfläche innerhalb des Gärtanks geschlossen wird. Durch das Schließen auf den Radius des Abbildes der Oberfläche kann auf die Entwicklung von Schaum in dem Gärtank geschlossen werden, wobei die Entwicklung von Schaum als ein Hinweis für die Belüftung während des Befüllens und des Fermentationsstatus, insbesondere das Ende der Gärung, dient.

Zur Bestimmung des Radius des Abbildes der Oberfläche werden vorteilhafterweise vom Abbild der Konusmitte ausgehende Strahlen oder Kreissegmente als bestimmte Bereiche bestimmt. Das Kriterium betrifft die Ableitung der Intensität entlang der Strahlen oder Kreissegmente. Das am weitesten außenliegende relative Maximum der Ableitung mit mindestens einem vorgegebenen Absolutwert der Ableitung ist ein Maß für den Radius des Abbildes der Oberfläche entlang einer spezifischen Richtung oder eines spezifischen Strahls oder Kreissegments. Auf den Radius des Abbildes der Oberfläche des Füllmediums in dem Gärtank wird durch Betrachtung der relativen Häufigkeit eines Wertes des Radius für verschiedene Strahlen geschlossen, wobei vorzugsweise darauf geschlossen wird, dass der zu ermittelnde Radius sich aus dem Maximalwert der relativen Häufigkeit eines Radiuswertes von verschiedenen Strahlen ergibt. Durch die Auswertung von dem Radius in verschiedene Richtungen kann bei hinreichender Anzahl von ausgewählten Richtungen oder Strahlen eine gute statistisch fundierte Aussage über den Radius erreicht werden, ohne dass einzelne Reflexe oder sonstige Bildstörungen zu einer starken Verfälschung des geschlossenen Radius führen.

Eine besonders vorteilhafte Ausführungsform der Erfindung besteht darin, dass die erfindungsgemäße Untersuchung in die Steuerung der Anlage einfließt. Hierbei können die gewonnenen Erkenntnisse über den Zustand in dem Gärtank die verschiedensten Prozessparameter, wie sie bei der Gärung von beispielsweise Bier, üblicherweise berücksichtigt werden, gesteuert bzw. geregelt werden.

Vorteilhaft ist auch ein Speichermedium, das ein Computerprogramm oder eine Befehlssequenz enthält, mit der das erfindungsgemäße Verfahren realisiert werden kann. Weiterhin ist eine rechnergesteuerte Vorrichtung, die Mittel enthält, die das erfindungsgemäße Verfahren ausführen, von Vorteil.

Im Folgenden werden Ausgestaltungen des erfindungsgemäßen Verfahrens mit Hilfe der anliegenden Figuren erläutert. Dabei zeigt:
- Fig.1: eine schematische Zeichnung eines Gärtanks,
- Fig.2: erstellte Bilder, sowie bestimmte Bereiche, und Pixel, die ein Kriterium erfüllen wie sie in dem erfindungsgemäßen Verfahren auftreten,
- Fig.3: erstellte Bilder, bestimmte Bereiche, Kriterien und eine Häufigkeitsverteilung von Radien wie sie in dem erfindungsgemäßen Verfahren vorkommen.
- Fig.4: schematische Zeichnungen zur Erläuterung von Bereichen die bestimmt wurden, Häufigkeitsverteilungen und Kriterien wie sie im erfindungsgemäßen Verfahren vorkommen.
- Fig.5: Flussdiagramm zur Veranschaulichung der erfindungsgemäßen Verfahrensschritte.

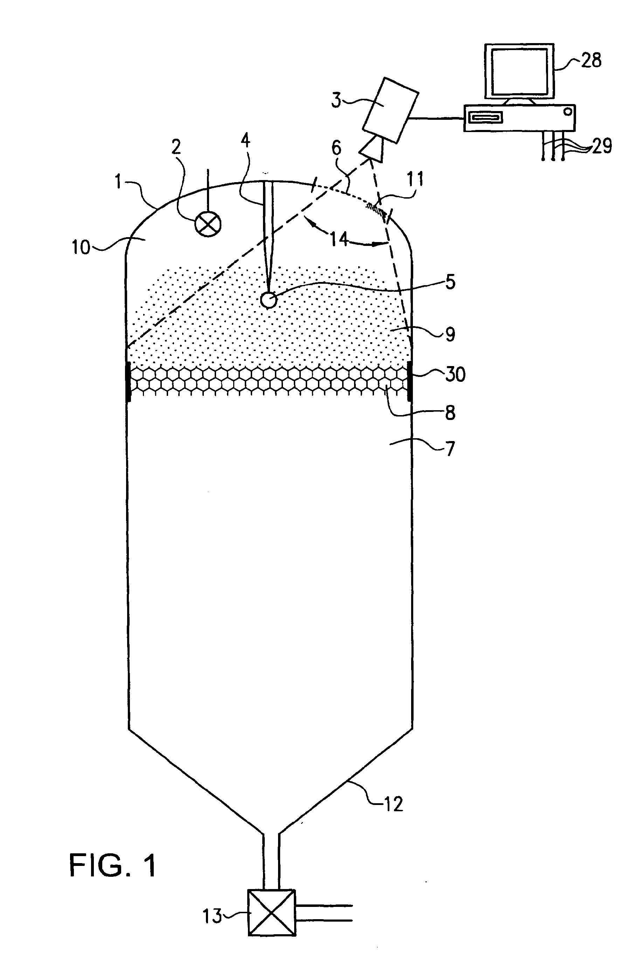

Fig. 1 zeigt einen Gärtank 1 mit einer Beleuchtung 2, der mit einem Medium 7 weitgehend gefüllt ist. Das Medium 7 kann beispielsweise gärende Bierwürze sein. Oberhalb der Bierwürze 7 befindet sich eine Schaumdecke 8. In dem Freiraum 10, oberhalb der Schaumdecke 8, ist Nebel 9 dargestellt, der nur zeitweise in dem Gärtank auftritt. Eine Schaumdecke 8 ist sicherlich nicht immer in einem derartigen Gärtank vorhanden. Der Brandthefebereich, in dem der Schaum Kontakt mit der Innenwand des Gärtanks hat,ist mit der Bezugsziffer 30 bezeichnet. Das Verfahren kann auch durchgeführt werden, falls das Medium 7 und/oder die Schaumdecke 8 fehlt und z.B. die Reinheit des Gärtanks untersucht werden soll.

Weiterhin ist eine Sprühkugel 5 zusammen mit einer Zuleitung zu der Sprühkugel 4 dargestellt. Auf einem Schauglas 6 ist ein Beschlag 11 dargestellt, der zeitweise auf einem derartigen Schauglas durch Kondensation von z.B. Wasser auftreten kann. Eine Kamera 3 ist so angeordnet, dass sie durch das Schauglas 6 hindurch in den Innenraum des Gärtanks 1 unter dem Blickwinkel 14 blickt. Am unteren Ende des Gärtanks ist ein Konus 12 dargestellt, wie er üblicherweise bei derartigen Gärtanks verwendet wird. Ein Ventil 13 schließt bzw. öffnet eine Zu- oder Ableitung des Gärtanks. Eine rechnergesteuerte Vorrichtung bzw. ein Rechner 28 enthält Mittel, die das erfindungsgemäße Verfahren ausführen. Weiterhin sind Signalausgänge 29 vorgesehen, über die die Erkenntnisse über den Zustand im Gärtank genutzt werden können, um die Gärung, die Reinigung etc. des Gärtanks zu steuern bzw. zu regeln.

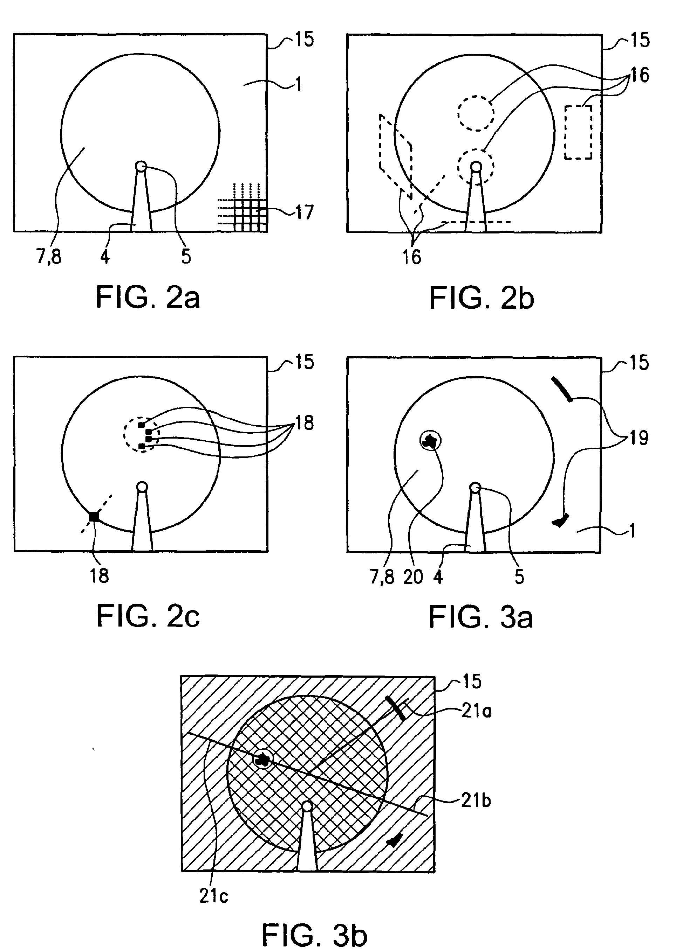

In Fig. 2 sind verschiedene Darstellungen zur Erläuterung des Verfahrens gezeigt. In Fig. 2a ist ein Bild 15 dargestellt, in dem die Oberfläche der Flüssigkeit 7 oder der Schaumdecke 8, die Sprühkugel 5 mit der Zuleitung 4 und die Innenseite des Gärtanks 1 zu erkennen ist. Rechts unten in Fig. 2a ist abschnittsweise und repräsentativ für das ganze Bild dargestellt, wie sich das Bild schematisch aus Pixeln (17) zusammensetzt. In Fig. 2b sind bestimmte Bereiche 16 dargestellt, wie sie erfindungsgemäß bestimmt werden. Sowohl linien- als auch kreis- und rechteckförmige als auch anders geformte bestimmte Bereiche sind zu erkennen. In Fig. 2c ist dargestellt, wie einzelne Pixel 18, die innerhalb der bestimmten Bereiche 16 liegen und die das Kriterium, welches das erfindungsgemäße Verfahren verwendet, erfüllen. Aus der Lage oder der Anzahl der Pixel 18 kann ein Maß festgestellt werden, das auf einen zu untersuchenden Zustand in dem Gärtank schließen lässt.

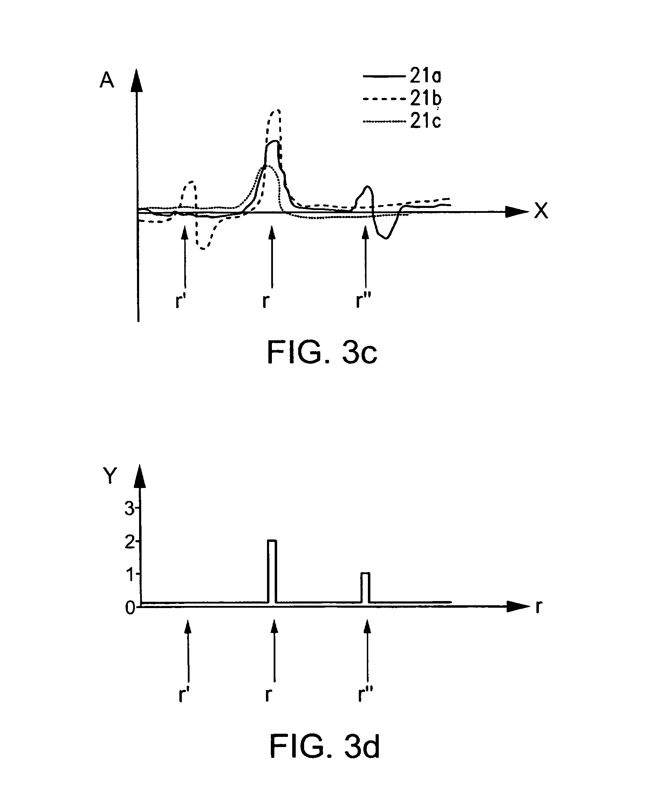

Ein spezielles erfindungsgemäßes Verfahren wird anhand der Fig. 3 erläutert. In Fig. 3a ist wieder ein erstelltes Bild gezeigt, in dem die Sprühkugel 5 und die Zuleitung 4 zu erkennen sind. Auf dem Bild ist weiterhin die Oberfläche der Flüssigkeit 7 oder der Schaumdecke 8 zu erkennen. Weiterhin ist ein Fleck 20 in dem Bild sowie Reflexe 19 eingezeichnet. Derartige Reflexe können von Schleifspuren oder Schweißnähten der Innenseite des Gärtanks herrühren. In Fig. 3b ist gezeigt, dass der äußere Bereich, der das Abbild der Innenseite des Gärtanks darstellt, unterschiedlich hell sein soll im Vergleich zu dem Bereich, der die Oberfläche der Flüssigkeit 7/Schaumdecke 8 wiedergibt. Dies spiegelt sich beispielsweise darin wieder, dass der I-Farbwert für den Bereich des Gärtanks sich deutlich von dem I-Farbwert des Bereichs der Oberfläche des Mediums unterscheidet. Alternativ kann auch jeder andere Farbwert statt des I-Farbwertes eingesetzt werden. Weiterhin sind die bestimmten Bereiche 21a, 21b, 21c dargestellt, die linienförmige Bereiche sind. Die bestimmten Bereiche 21a bis 21c haben einen gemeinsamen Ursprung, der etwa in der Mitte des Abbildes der Oberfläche der Flüssigkeit 7/Schaumdecke 8 liegt.

Die Intensität wird also in dem inneren Bereich der Linien nahe dem Ursprung der Linien einen unterschiedlichen Wert haben im Vergleich zu dem äußeren Bereich, in dem die Linien sich im Bereich des Abbildes der Innenseite des Gärtanks befinden. Bei dem Übergang der Linie von innen nach außen, d.h. von z.B. einem dunklen zu einem helleren Bereich, wird sich eine Intensitätssteigerung ergeben, die in der Ableitung der Intensität wiedergefunden werden kann. Die Ableitung der Intensität ist in Fig. 3c für die drei Linien 21a bis 21c dargestellt. Vor Bilden der Ableitung ist eine rechnerische Glättung der abzuleitenden Kurve vorteilhaft. Der Koordinatenursprung bezieht sich dabei auf das Linienende, das in der Bildmitte in Fig. 3b liegt. Der umgedrehte Liniensinn, dass heißt, dass der Koordinatenursprung am äußeren Ende der Linie liegt, ist ebenfalls möglich. Dadurch würde hohe x-Werte in Figur 3c einem Pixel nahe des Zentrums des Bildes entsprechen. Die Ableitung der Intensität der Linie 21b, die gestrichelt dargestellt ist, zeigt lediglich ein Maximum bei einem Wert, der mit r markiert ist. Die Linien 21a und 21c, die durch Störungen 19,20 im Bild der Fig. 3b laufen, zeigen im Weiteren Maxima bei r' und r". In Fig. 3d ist aufgetragen, mit welcher Häufigkeit y die verschiedenen r-Werte, bei denen ein Maximum in der Ableitung A auftritt, vorkommen. Hierbei ist zu beachten, dass nur jeweils dasjenige Maximum betrachtet wird, das den größten x-Wert für eine Linie hat. Das Kriterium zur Analyse eines Pixels ist somit, dass die Ableitung an der Stelle des Pixels einen gewissen Minimalwert überschreitet und dass das Pixel von denjenigen Pixeln, an denen die Ableitung den Minimalwert überschreitet dasjenige ist, das am weitesten außen (d.h. mit dem größten x-Wert) liegt. (Entsprechendes gilt für den kleinsten x-Wert, falls der Richtungssinn der Linien umgedreht ist, s.o..)

Wie in Fig. 3d dargestellt, ist dasjenige Pixel, bei dem die Ableitung einen Minimalwert überschreitet und für eine Linie am weitesten außen liegt und den Wert r hat, zweimal vorgekommen und der Wert r" einmal. Entsprechend ergibt sich eine erhöhte Häufigkeit bei dem Wert r. Somit wird geschlossen, dass der Zustand in dem Gärtank derjenige ist, bei dem das Abbild der Oberfläche mit einem Radius r erscheint. Aus dem Radius r ergibt sich die Füllstandshöhe des Mediums in dem Gärtank bzw. die Höhe der Oberfläche des Mediums. Alternativ ist es auch möglich nicht den r-Wert bei dem Maximum der Ableitung zu nehmen, sondern bei dem mit einem Maximum verbundenen Nulldurchgang der Ableitung. Dieser liegt bei r' und r" bei etwas größeren Werten als das Maximum der Ableitung.

Ebenfalls ist es vorteilhafterweise möglich mehr bzw. wesentlich mehr Linien als 3 Linien zur Bestimmung des Radiuses zu verwenden.

Das in Fig. 3 beschriebene Verfahren kann beispielsweise dazu eingesetzt werden, bei einer vorgegebenen Flüssigkeitsmenge auf die Höhe der Schaumdecke zu schließen. Die Höhe der Schaumdecke verändert sich in charakteristischer Weise während des Gärvorgangs. Hierbei ist es somit vonnöten, zu verschiedenen Zeiten auf den Radius des Abbildes der Oberfläche zu schließen und somit den zeitlichen Verlauf des Radius zu erhalten. Nimmt der Radius ab, d.h., sinkt die Oberfläche der Schaumdecke ab, so kann dies als Hinweis darauf gewertet werden, dass die Gärung dem Ende zugeht, und somit kann die Wahrscheinlichkeit für die Aussage, dass die Gärung zu Ende ist, erhöht werden.

Ebenfalls wäre es möglich, zu schließen, dass die geschlossene Wahrscheinlichkeit für den Zustand des Überweissens bei abnehmendem Radius erniedrigt ist.

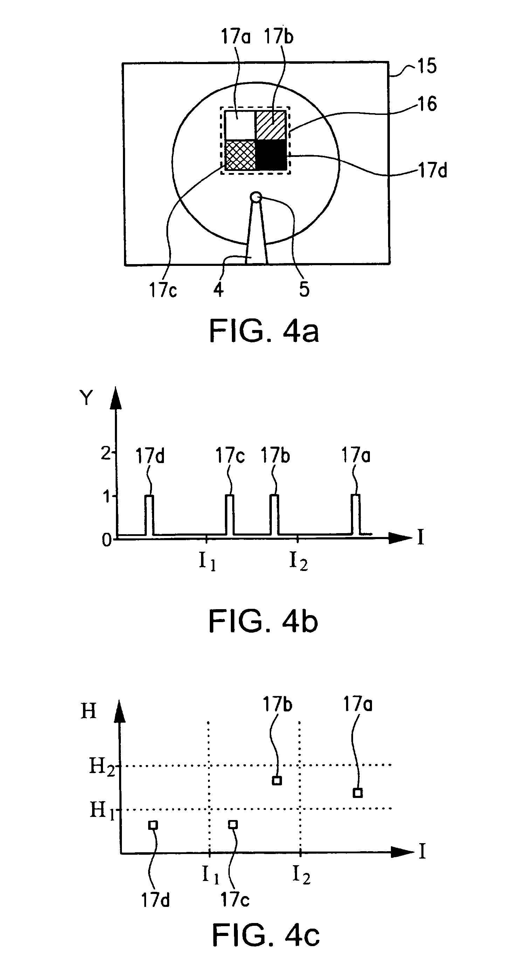

In Fig. 4 ist eine weitere erfindungsgemäße Verfahrensweise beschrieben. In Fig. 4a ist ein Bild 15 gezeigt, in dem ein bestimmter Bereich 16 bestimmt ist. In dem bestimmten Bereich 16 sind vier Pixel 17a, 17b, 17c, 17d stark vergrößert dargestellt. Die verschiedene Schattierung der Pixel 17a bis 17d soll auf mögliche Unterschiede der RGB- oder HSI-Farbwerte hinweisen. Beispielsweise wird in der folgenden Erläuterung angenommen, dass die Pixel 17a bis 17d eine abnehmende Intensität (I) haben sollen. Da in dem erfindungsgemäßen Verfahren nur die Pixel innerhalb des bestimmten Bereiches 16 analysiert werden, werden im Folgenden nur die vier Pixel 17a bis 17d exemplarisch weiterbehandelt. In Fig. 4b ist die Häufigkeit y dargestellt, mit der Pixeln mit einer Intensität 1 auftreten. Da nur vier Pixel betrachtet werden, die verschiedene Intensitäten haben sollen, ist die Häufigkeit des Auftretens einer Intensität in diesem Fall immer 1, da für jede Intensität genau ein Pixel vorhanden ist. Ein Kriterium zum Analysieren der Pixel könnte hierbei beispielsweise lauten, dass die Intensität der Pixel zwischen dem Wert I₁ und I₂ liegen soll. Auch wäre es denkbar, als Kriterium anzugeben, dass die Pixel eine Intensität größer I₁ oder kleiner I₂ haben sollen oder eine Intensität kleiner I₁ oder größer I₂ oder eine Intensität kleiner I₁ oder eine Intensität größer I₂ haben sollen. Weiterhin muss nicht zwingenderweise die Intensität betrachtet werden, sondern jeder andere beliebige Farbwert, wie z.B. auch die Farbigkeit, oder die Ableitung der Intensität bei einem Pixel kann zur Vorgabe eines vergleichbaren Kriteriums herangezogen werden.

Wird als Kriterium vorgegeben, dass die Intensität der Pixel zwischen I₁ und I₂ liegen soll, so erfüllen die Pixel 17c und 17b das Kriterium. Somit kann als Maß festgestellt werden, dass 50 % der Pixel innerhalb des bestimmten Bereiches 16 das Kriterium erfüllen. Das festgestellte Maß könnte z.B. mit 50 entsprechend 50 % oder mit 0,5 angegeben werden. In Fig. 4c ist eine weitere Möglichkeit gezeigt, Kriterien vorzugeben, mit denen die Pixel in dem bestimmten Bereich 16 analysiert werden sollen. In einem zweidimensionalen Feld ist auf einer Achse die Intensität I und auf einer zweiten Achse senkrecht dazu der Farbwert H (Hue) aufgetragen. Die vier Pixel 17a bis 17d sind exemplarisch in dem zweidimensionalen Koordinatensystem eingetragen. Durch Kombination der I- und der H-Farbwerte lassen sich Kriterien reichhaltiger Art vorgeben. Beispielsweise ist es möglich, als Kriterium vorzugeben, dass die Intensität zwischen I₁ und I₂ und der H-Farbwert zwischen H₁ und H₂ liegen soll. Dieses Kriterium würde nur von dem Pixel 17b erfüllt. Das Maß, das sich feststellen ließe, wäre somit 25 % (= 0,25). Wäre als Kriterium vorgegeben, dass der H-Farbwert < H₁ und die Intensität < I₂ sein soll, so würden die Pixel 17c und 17d das Kriterium erfüllen, und das festgestellte Maß wäre somit 50 % (0,5).

Alternativ ist es auch möglich, anstatt der relativen Häufigkeit der Pixel, die innerhalb eines bestimmten Bereiches 16 ein Kriterium erfüllen, die absolute Zahl der Pixel als Maß festzustellen. In dem letzten Beispiel wäre somit das festgestellte Maß 2, da die Pixel 17b und 17d das Kriterium erfüllen. Neben den H- und I-Farbwerten können auch die S-Farbwerte und/oder jeder der RGB-Farbwerte zur Bildung von weiteren Kriterien herangezogen werden, wobei, da, wie in Fig. 4C, zwei Farbwerte ausgewertet werden, auch drei, vier, fünf oder noch mehr Farbwerte in das Kriterium einfließen.

Das Kriterium der Übersteuerung kann auch anhand von Fig. 4b und 4c erläutert werden. Das Kriterium der Übersteuerung besagt, dass ein Pixel einen I-Farbwert größer als einen vorgegebenen Wert aufweist. Dieser vorgegebene Wert sollte relativ weit oben in der möglichen Skala der I-Farbwerte liegen, d.h. etwa bei 90 % des maximal möglichen I-Farbwertes. Sei der Wert, oberhalb dessen ein Pixel als übersteuert bezeichnet wird, der Wert I₂, so erfüllt das Pixel 17a das Kriterium der Übersteuerung.

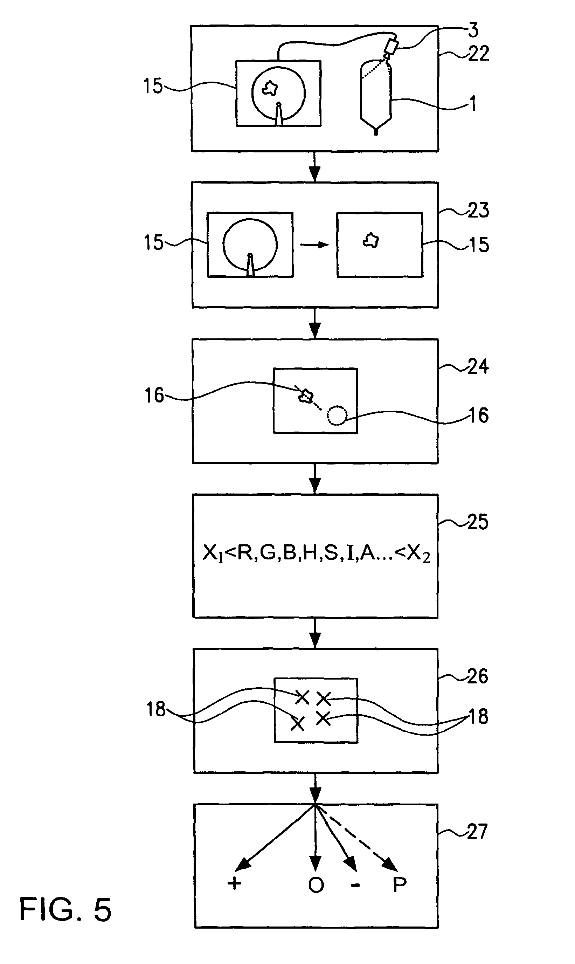

Die Fig. 5 ist der zeitliche Ablauf, mit dem ein erfindungsgemäßes Verfahren durchgeführt werden kann, schematisch dargestellt. Zunächst wird in Schritt 22 ein Bild mit einer Kamera 3 vom Inneren eines Gärtanks 1 aufgenommen. Das aufgenommene Bild wird im Schritt 23 weiterbearbeitet. Hierbei ist es beispielsweise möglich, das Bild scharf zu zeichnen oder ein Referenzbild abzuziehen. Mit dem Schritt 23 wird ein Bild erstellt. Weiterhin ist es auch möglich, in dem Schritt 23 keine Operation an dem aufgenommenen Bild vorzunehmen, so dass das Erstellen des Bildes gleich dem Aufnehmen des Bildes ist.

In Schritt 24 werden Bereiche 16 des Bildes bestimmt (gestrichelt dargestellt). Im Folgenden werden somit nur die Pixel, die innerhalb des bestimmten Bereiches liegen, betrachtet.

In Schritt 25 wird ein Kriterium vorgegeben, das beispielsweise den RGB- oder HSI-Farbwert zur Bildung eines Kriteriums enthält. Auch ist es möglich, die Farbigkeit, die Übersättigung oder die Ableitung bei einem Pixel heranzuziehen, um ein Kriterium zu bilden. Das Kriterium kann beispielsweise darin bestehen, dass ein Farbwert oder ein sonstiger Wert, der für ein Pixel eine charakteristische Aussage ermöglicht, über, unter oder zwischen Werten X₁ und X₂ liegen. Auch die Differenzen von Farbwerten zu anderen Pixeln können zur Bildung von Kriterien herangezogen werden. Das Kriterium kann auch nur in der Angabe einer charaktersitischen Größe z.B. der Farbigkeit liegen. Nach Vorgabe des Kriteriums 25 werden anschließend die Pixel, die innerhalb eines bestimmten Bereiches 16 liegen und das Kriterium aus Schritt 25 erfüllen, ermittelt. Daraus ergibt sich ein Maß, das festgestellt wird. Das Maß kann sich hierbei erfindungsgemäß aus der Lage der Pixel, aus der Anzahl der Pixel bzw. der relativen Anzahl der Pixel, d.h., bezogen auf die Anzahl der Pixel in dem bestimmten Bereich 16, als auch aus der Stärke mit der ein Kriterium gegeben ist (z.B. die Stärke der Farbigkeit) ergeben. Aus dem festgestellten Maß aus Schritt 26 wird in Schritt 27 ein Schluss gezogen, der beispielsweise darin bestehen kann, dass ein Zustand gut (+), zufriedenstellend (0) oder schlecht (-) ist. Auch eine Einteilung in lediglich gut (+) und schlecht (-) ist möglich. Alternativ ist es ebenfalls möglich, das Vorhandensein eines zu untersuchenden Zustandes (+) bzw. auf das Nichtvorhandensein (-) eines zu untersuchenden Zustandes zu schließen.
Alternativ ist es ebenfalls möglich, statt direkt auf das Vorhandensein eines Zustandes zu schließen, auf eine Wahrscheinlichkeit P für das Vorliegen eines Zustandes zu schließen.

Die Schritte 24 bis 27 können auch anhand eines erstellten Bildes mehrmals wiederholt werden. So ist es beispielsweise möglich, erst zu untersuchen, ob das Schauglas verschmutzt ist oder ob Nebel in dem Tank vorliegt und wenn in den beiden Fällen geschlossen wird, dass dies nicht der Fall ist, so kann, beginnend mit dem Schritt 24, eine weitere Untersuchung auf einen weiteren Zustand in dem Gärtank beginnen. Auch ist es möglich, die Schritte 24 bis 27 wiederholt zum Feststellen verschiedener Maße durchzuführen, um durch Erhalten von verschiedenen Wahrscheinlichkeiten auf eine Gesamtwahrscheinlichkeit für das Vorliegen eines Zustandes zu schließen. Auch kann nach einmaligen Durchführen des erfindungsgemäßen Verfahrens mit der Erstellung eines neuen Bildes begonnen werden.

Im Folgenden sollen beispielhaft verschiedene Zustände, auf die das Innere eines Gärtanks untersucht werden kann, zusammen mit den entsprechenden Kriterien und den Maßen, die festgestellt werden können, dargestellt werden.

Zu Untersuchungen des Zustandes des Schauglases wird beispielsweise ein Bild durch Aufnehmen erstellt. Die Bereiche, die bestimmt werden, sind Paare von Linien, die parallel zueinander verlaufen und durch ein Gebiet in dem Bild gehen, indem bei einem sauberen Schauglas starke Differenzen der Helligkeitsverläufe (I-Farbwert) auftreten. Die Linien liegen im Vergleich zu ihrer Länge sehr dicht beieinander. In dem Bereich, in dem die Zuleitung 4 zu der Sprühkugel 5 in dem Bild auftreten, sind starke Differenzen der Helligkeitsverläufe gegeben. Den Pixel einer Linie eines Linienpaares werden die Helligkeitsverläufe der anderen Linien des Linienpaares abgezogen. Dadurch, dass die Kanten der Zuleitung 4 zur Bildmitte hin näher zusammenrücken, ergibt sich bei der Differenzbildung der Intensitätsfarbwerte stark ausgeprägte Kanten in den Intensitätsdifferenzkurven, die sich in starken Maxima und Minima ausprägen. Falls die Maxima bzw. Minima vorgegebene Minimalwerte bzw. Maximalwerte über- bzw. unterschreiten, so erhöht sich das festgestellte Maß mit jedem Über- bzw. Unterschreiten einer Linie.

Das Kriterium ist derart formuliert, dass ein Pixel das Kriterium erfüllt, falls der I-Farbwert des Pixels einen Helligkeitsunterschied zu dem entsprechenden Pixel der anderen Linie des Linienpaares aufweist, der Helligkeitsunterschied einen Minimal- bzw. Maximalwert über- bzw. unterschreitet und bisher noch bei keinem anderen Pixel derselben Linie das Über- bzw. Unterschreiten festgestellt wurde. Für jede Linie kann somit das Überschreiten nur einmal festgestellt werden und entsprechendes gilt für das Unterschreiten.

Das festgestellte Maß ist hierbei die Summe der Pixel, die ein Über- bzw. Unterschreiten aufweisen. Der Maximalwert für das festgestellte Maß ist somit zweimal die Anzahl der Linienpaare, da für jedes Linienpaar für eine Linie einmal das Über und einmal das Unterschreiten gefunden werden kann. Aus dem Verhältnis des festgestellten Maßes zu dem maximal möglichen Wert (zweimal die Anzahl der Linienpaare) lässt sich auf den Zustand des Schauglases schließen. Ist das festgestellte Maß etwa größer als 60 % des maximal möglichen Wertes des festgestellten Maßes, kann davon ausgegangen werden, dass das Schauglas nicht verschmutzt ist.

Als nächstes soll dargelegt werden, wie das erfindungsgemäße Verfahren verwendet wird, um den Zustand von Nebel im Gärtank zu untersuchen. Zunächst wird ein Bild aufgenommen und anschließend scharfgezeichnet. Anschließend werden Bereiche bestimmt, die Linien sind und bei denen, falls kein Nebel im Tank vorliegt, scharfe Übergänge von Hell nach Dunkel bzw. von Dunkel nach Hell auftreten. Das Kriterium, anhand dessen die Pixel in dem bestimmten Bereich des Bildes analysiert werden, ist folgendermaßen formuliert:
Falls die Ableitung des I-Farbwertes entlang der Linie bei einem Pixel einen Absolutwert unter einem vorgegebenen Schwellwert aufweist, so erfüllt das Pixel das Kriterium. Mit Absolutbetrag ist derjenige Wert gemeint, der sich durch Multiplikation mit -1 ergibt, falls der Wert negativ ist, und der Wert selber, falls er positiv ist. Falls alle Pixel einer Linie das Kriteriums erfüllen so wird das festzustellende Maß vorläufig um eins erhöht. Nach Prüfen aller Linien ergibt sich das festgestellte Maß aus dem vorläufig festgestellten Maß. Liegt das festgestellte Maß, über einem vorgegeben Wert von etwa 80 bis 99 % des maximal möglichen festgestellten Maßes, so kann daraus geschlossen werden, dass der Zustand, Nebel im Tank, vorliegt.

Im Folgenden soll erläutert werden, wie der Reinigungszustand des Gärtanks untersucht werden kann. Ein Bild wird durch Aufnehmen eines Bildes erstellt, und der Bereich der bestimmt wird, ist der Bereich außerhalb eines vorgegebenen Kreises, wobei der bestimmte Bereich denjenigen Bildbereich umfasst, in dem abgesetzte Brandthefe erscheint. Die Brandthefe bildet sich vorwiegend in dem Bereich, in dem der Schaum 8 Kontakt mit der Innenwand des Gärtanks 1 hat. Wie aus Fig. 1 zu erkennen, wird dies im Wesentlichen den Außenbereiche des Bildes betreffen, das die Kamera dabei aufnimmt. Als Kriterium wird definiert, dass der H-, S- und I-Farbwert in einem jeweils für jeden Farbwert individuell vorgegebenen Intervall liegt, der die typisch gelblich-bräunliche Brandthefefarbe spezifiziert. Für nur zwei statt drei Farbwerte ist dies schematisch in Figur 4c dargestellt. Das festgestellte Maß ist die Anzahl der Pixel, die in dem bestimmten Bereich das Kriterium erfüllen. Auch hierbei ist es wieder möglich, ein relatives oder absolutes Maß festzustellen, je nachdem ob die Anzahl der Pixel, die das Kriterium erfüllen, auf die Anzahl der Pixel, die in dem bestimmten Bereich liegen, normiert wird. Liegt die Anzahl der Pixel, die das Kriterium erfüllen, unterhalb eines vorgegebenen Schwellwerts, so kann geschlossen werden, dass der Tank sauber ist.

Eine weitere Möglichkeit, das erfindungsgemäße Verfahren einzusetzen, besteht in der Untersuchung der Belüftung während des Befüllens. Durch eine gute Belüftung bildet sich eine feine, cremige Schaudecke 8 aus. Auch hier wird durch Aufnehmen ein Bild erstellt. Als Bereich 16 wird der Bereich bestimmt, der bei einem leeren Gärtank das Abbild des Konus aufweist. Bei einem zylindrischkonischen Gärtank, wie in Fig. 1 dargestellt, erscheint dieser Bereich kreisförmig, etwa in der Bildmitte. Hierbei werden für die RGB-Farbwerte Intervalle vorgegeben, innerhalb derer die Farbwerte R,G und B liegen sollen. Auch die Betrachtung von nur ein oder zwei Farbwerten ist möglich.

Das Kriterium ist so formuliert, dass der R,G und B-Farbwert eines Pixels in je einem Intervall liegen muss (wie dies schematisch für den H- und I-Farbwert in Fig. 4c dargestellt ist). Das festgestellte Maß ist auch hierbei die Anzahl der Pixel, die innerhalb des bestimmten Bereiches das Kriterium erfüllen. Durch Festlegen eines Schwellwertes von z.B. 50% des maximal möglichen Werts kann aus dem festgestellten Maß auf die Güte der Belüftung während des Befüllens in dem Gärtank geschlossen werden. Liegt also der Anteil der Pixel, die das Kriterium erfüllen, oberhalb des Schwellenwertes, so kann auf den Zustand einer guten Belüftung geschlossen werden.

Durch Vorgeben eines Kriteriums mittels Intervallen für die H-, S- und I-Farbwerte und Vorgeben eines Schwellwertes für die Anzahl der Pixel, die das Kriterium erfüllen, kann auf einen schaumlosen Fleck in der Mitte der Oberfläche des Füllmediums geschlossen werden. Der bestimmte Bereich hierbei ist ein Kreis der das Zentrum des Abbildes des Konuses umfasst, jedoch nicht den gesamten Bereich des Abbildes des Konuses. Das Merkmal des schaumlosen Flecks in der Mitte ist ein Merkmal für das Ende der Gärung.

Durch Vorgeben von einem Intervall für den I-Farbwert als Kriterium für Pixel und Vorgeben eines Schwellwertes für den Anteil der Pixel, die innerhalb des bestimmten Bereiches das Kriterium erfüllen, kann auf Schaum im Konusbereich geschlossen werden, welcher ein weiteres Merkmal für das Ende der Gärung ist. Hierbei ist der bestimmte Bereich derjenige Bereich, der bei einem leeren Tank ein Abbild des Konus aufweist.

Wird als Kriterium die Übersteuerung von Pixeln vorgegeben und ein Schwellwert für den Anteil der Pixel, die übersteuert sind, vorgegeben, so lässt sich auf Reflexionen im Konusbereich schließen (bestimmter Bereich = Bereich des Abbildes des Konus), die ein Merkmal für das Ende der Gärung sind. Die drei Merkmale des schaumlosen Flecks in der Mitte, des Schaumanteils im Konus und der Reflexionen im Konus lassen sich zusammenfassen, um eine abschließende Wahrscheinlichkeit dafür anzugeben, dass die Gärung zu Ende ist. Hierbei lässt sich festhalten, dass, wenn zumindest zwei Merkmale erfüllt sind, geschlossen wird, dass die Gärung abgeschlossen ist.

Der Zustand des Überweissens kann dadurch untersucht werden, dass auf die Wahrscheinlichkeit für den Zustand des Ende der Gärung geschlossen wird und sich die Wahrscheinlichkeit für den Zustand des Überweissens aus 1 minus der Wahrscheinlichkeit für den Zustand des Endes der Gärung ergibt.

Hierbei kann weiterhin auch das Schließen auf einen Radius erfolgen, wobei dies vorteilhafterweise mehrmals passiert und die Wahrscheinlichkeit für das Vorliegen des Überweissens erniedrigt, falls der Radius über einen gewissen Zeitraum mehr als einen substantiellen Anteil (etwa 5 bis 10 %) abgenommen hat.

Der Zustand der Hauptgärung kann dadurch untersucht werden, dass auf den Radius zu verschiedenen Zeiten geschlossen wird. Ein zunehmender Radius ist ein Signal dafür, dass die Hauptgärung eintritt.

Der Zustand des Eintauchens der Sprühkugel in beispielsweise den Schaum 8 kann dadurch untersucht werden, dass durch Aufnehmen ein Bild erstellt wird, ein Bereich bestimmt wird, der das Abbild der Sprühkugel beinhaltet oder zumindest zum Teil beinhaltet, und als Kriterium die Übersteuerung von Pixeln vorgegeben wird. Der Anteil der übersteuerten Pixel ist hierbei das Maß, das festgestellt wird, um auf den Zustand des Eintauchens der Sprühkugel zu schließen. Durch Vorgabe eines Schwellwertes kann auf das Eintauchen oder auf das kurz bevorstehende Eintauchen geschlossen werden.

Zur Untersuchung des Leerseins des Gärtanks können drei verschiedene Merkmale untersucht werden. Auf jedes Merkmal wird mit dem erfinderischen Verfahren einzeln geschlossen. Für das erste Merkmal wird das Bild durch Aufnehmen eines Bildes und Abziehen eines Referenzbildes erstellt. Das Referenzbild ist hierbei von einem leeren und sauberen Tank aufgenommen worden. Das Kriterium, welches vorgegeben wird, ist ein Intervall für die I-Farbwerte. Der bestimmte Bereich ist ein zentraler Bereich des Bildes, wobei der Brandthefebereich ausgelassen ist. Das festgestellte Maß ist hierbei die Anzahl der Pixel, deren I-Farbwert in dem vorgegebenen Intervall liegt. Durch Vorgabe eines Schwellwertes kann auf das erste Merkmal für das Leersein des Gärtanks geschlossen werden.

Für das zweite Merkmal für den Zustand des Leerseins des Gärtanks ist das Kriterium die Farbigkeit der Pixel und der bestimmte Bereich derselbe wie der des ersten Merkmals für den Zustand des Leerseins des Gärtanks. Durch Vorgabe eines Schwellwerts der Farbigkeit kann auch hier auf das Leersein des Tanks geschlossen werden. Ein drittes Merkmal zur Untersuchung des Zustandes des Leerseins des Gärtanks gibt als bestimmten Bereich den Bereich vor, der bei einem leeren Gärtank das Abbild des Konuses wiedergibt. Das Kriterium ist die Übersteuerung der Pixel, und das festgestellte Maß ist die Anzahl der Pixel, die übersteuert sind. Durch Vorgabe eines Schwellwertes lässt sich auf ein weiteres Merkmal für den Zustand des Leerseins des Gärtanks schließen.

Durch Zusammenfassung der drei Merkmale für den Zustand des Leerseins des Gärtanks kann abschließend auf den Zustand des Leerseins des Gärtanks geschlossen werden. Nur falls alle drei Merkmale erfüllt sind, wird auf den Zustand des Leerseins des Gärtanks geschlossen.

## Patentansprüche

1. Verfahren zur Untersuchung mindestens eines Zustands in einem Behältnis (1), wie es bei der Getränkeherstellung bei der Vermehrung und/oder Vergärung von/mit Organismen verwendet wird, wie z.B. einem Gärtank, Hefereinzuchttank, biologischen Milchsäuretank, Lagertank oder dergleichen, insbesondere einem Gärtank (1) für Bier, das die folgenden Schritte umfasst:
- Erstellen (22,23) mindestens eines Bildes mit Pixeln,
- Bestimmung (24) mindestens eines Bereiches (16) des Bildes (15) durch Auswahl von Pixeln (17),
- Vorgeben (25) von mindestens einem Kriterium, an Hand dessen die Pixel (17) in dem bestimmten Bereich (16) des Bildes analysiert werden,
- Feststellen (26) mindestens eines Maßes aus der Lage der Pixel (18) und/oder aus der Anzahl der Pixel (18), die das mindestens eine Kriterium erfüllen und innerhalb des bestimmten Bereiches (16) liegen und/oder dem Verhältnis der Anzahl der Pixel (18), die das mindestens eine Kriterium erfüllen und innerhalb des bestimmten Bereiches (16) liegen zu der Anzahl der Pixel (17,18), die innerhalb des bestimmten Bereiches liegen, und/oder der Stärke mit der Pixel ein Kriterium erfüllen, und
- Schließen (27) auf den Zustand in dem Behältnis unter Berücksichtigung des festgestellten Maßes.

2. Verfahren nach Anspruch 1 **dadurch gekennzeichnet, dass** das Behältnis (1) eine zylindro-konische Form mit mindestens einem Konus (12) hat.

3. Verfahren nach einem der Ansprüche 1 oder 2, **dadurch gekennzeichnet, dass** ein Kriterium den R- und/oder G- und/oder B-Farbwert in RGB Farbsystem betrifft.

4. Verfahren nach einem der Ansprüche 1 bis 3, **dadurch gekennzeichnet, dass** ein Kriterium den H- und/oder S- und/oder I Farbwert im HSI-Farbsystem betrifft.

5. Verfahren nach einem der Ansprüche 1 bis 4, **dadurch gekennzeichnet, dass** ein Kriterium die Farbigkeit und/oder die Übersteuerung betrifft.

6. Verfahren nach einem der Ansprüche 1 bis 5, **dadurch gekennzeichnet, dass** das Bild (15) durch Aufnehmen erstellt wird.

7. Verfahren nach einem der Ansprüche 1 bis 6, **dadurch gekennzeichnet, dass** das Bild (15) dadurch erstellt wird, dass ein Bild (15) aufgenommen (22) und ein Referenzbild von diesem insbesondere pixelweise abgezogen wird (23).

8. Verfahren nach einem der Ansprüche 6 oder 7, **dadurch gekennzeichnet, dass** das Bild (15) von oben in Richtung des unteren Teils (12) des Behältnisses (1) aufgenommen wird.

9. Verfahren nach einem der Ansprüche 1 bis 8, **dadurch gekennzeichnet, dass** der bestimmte Bereich (16) des Bildes (15) das Abbild des Konuses (12) oder zumindest einen Teil des Abbildes des Konuses (12) umfasst bzw. aus dem Abbild des Konuses (12) besteht.

10. Verfahren nach einem der Ansprüche 1 bis 9, **dadurch gekennzeichnet, dass** der bestimmte Bereich (16) des Bildes (15) das Abbild mindestens eines Sprühkopfes (5) oder zumindest einen Teil des Abbildes des Sprühkopfs (5) umfasst bzw. aus dem Abbild mindestens eines Sprühkopfes (5) besteht.

11. Verfahren nach einem der Ansprüche 1 bis 10, **dadurch gekennzeichnet, dass** der bestimmte Bereich (16) des Bildes das Abbild des Brandthefebereichs (30 )oder zumindest einen Teil des Abbildes des Brandthefebereichs (30) umfasst bzw. aus dem Abbild des Brandthefebereichs (30) besteht.

12. Verfahren nach einem der Ansprüche 1 bis 11, **dadurch gekennzeichnet, dass** der bestimmte Bildbereich (16) einen oder mehrere einzelne Teilbildbereiche umfasst.

13. Verfahren nach einem der Ansprüche 1 bis 12 **dadurch gekennzeichnet, dass** der bestimmte Bildbereich (16) oder zumindest ein Teilbildbereich kreisförmig, linienförmig, quadratisch, rechteckig, n-eckig, oder unregelmäßig geformt ist.

14. Verfahren nach einem der Ansprüche 1 bis 13 **dadurch gekennzeichnet, dass** der bestimmte Bildbereich (16) oder zumindest ein Teilbildbereich durch zumindest Weglassen eines kreisförmig, linienförmig, quadratisch, rechteckig, n-eckig, oder unregelmäßig geformten Bildgebiets gegeben ist.

15. Verfahren nach einem der Ansprüche 1 bis 14, **dadurch gekennzeichnet, dass**
- der Zustand des Schauglases (6), und/oder
- der Zustand von Nebel im Behältnis (1), und /oder
- der Reinigungszustand des Behältnisses (1), und/oder
- der Zustand der Befüllung, und/oder
- der Zustand der Höhe der Oberfläche eines Medium (7,8) innerhalb des Behältnisses (1), und/oder
- der Zustand der Belüftung, und/oder
- der Zustand des Eintauchens einer Sprühkugel (5) in ein Medium (7,8) innerhalb des Behältnisses (1), und/oder
- der Zustand des Leerseins des Behältnisses (1), und/oder
- der Zustand der Fermentation, wie
- der Zustand des Endes der Gärung, und/oder
- der Zustand des Überweissens, und/oder
- der Zustand der Hauptgärung
untersucht wird.

16. Verfahren nach einem der Ansprüche 1 bis 15 **dadurch gekennzeichnet, dass** der Schluss (27) auf den Zustand in dem Behältnis (1) in Form eines Feststellens des Vorliegens eines Zustandes oder einer Wahrscheinlichkeit für das Vorliegen eines Zustands erfolgt.

17. Verfahren nach einem der Ansprüche 1 bis 16 **dadurch gekennzeichnet, dass** das Über- bzw. Unterschreiten des festgestellten Maßes über- bzw. unterhalb eines vorgegebenen Wertes den Schluss (27) auf den zu untersuchenden Zustand ergibt.

18. Verfahren nach einem der Ansprüche 16 oder 17 **dadurch gekennzeichnet, dass** die Wahrscheinlichkeit für das Vorliegen eines Zustandes gleich dem festgestellten Maß ist.

19. Verfahren nach einem der Ansprüche 1 bis 18 **dadurch gekennzeichnet, dass** die Untersuchung auf einen Zustandes in dem Behältnis (1) mit einem einzelnen Bild (15) erfolgt.

20. Verfahren nach einem der Ansprüche 1 bis 19 **dadurch gekennzeichnet, dass** die Untersuchung auf den Zustand in dem Behältnis (1) mit mehreren aufeinanderfolgenden Bildern (15) und insbesondere auch deren zeitlichen Abfolge bzw. Abstand erfolgt.

21. Verfahren nach einem der Ansprüche 1 bis 20 **dadurch gekennzeichnet, dass** die Koordinaten und/oder der Radius des Konuses (12) und/oder einer Sprühkugel (5) vorgegeben sind.

22. Verfahren nach einem der Ansprüche 1 bis 21 **dadurch gekennzeichnet, dass** der Kontrast und die Helligkeit, mit der das Bild (15) aufgenommen wird, vorgegeben sind.

23. Verfahren nach einem der Ansprüche 14 bis 22 **dadurch gekennzeichnet, dass** der Zustand des Schauglases (6) durch Bestimmung eines Bildbereiches (16) untersucht wird, in dem bei einem sauberen und durchsichtigen Schauglas (6) ein hoher Intensitätsunterschied vorzugsweise im Bereich des Abbildes eines Sprühkopfs (5) oder einer Zuleitung (4) zu dem Sprühkopf (5) auftritt.

24. Verfahren nach Anspruch 23 **dadurch gekennzeichnet, dass** das Kriterium den Intensitätsunterschied zwischen den Intensitäten der Pixel (17) der Linien (16) eines Linienpaares für eine vorbestimmte Zahl von Linienpaaren betrifft.

25. Verfahren nach Anspruch 24 **dadurch gekennzeichnet, dass** falls die Stärke des Intensitätsunterschiedes für ein Pixel (17) einen vorbestimmten Wert über- bzw. unterschreitet und kein anderes Pixel (17) derselben Linie (16) den vorbestimmten Wert über- bzw. unterschreitet das Kriterium von einem solchen Pixel (17) erfüllt ist.

26. Verfahren nach einem der Ansprüche 1 bis 25 **dadurch gekennzeichnet, dass** zur Erstellung (23) eines Bildes (15) eine Schärfungsoperation eines aufgenommenen Bildes (15) durchgeführt wird.

27. Verfahren nach einem der Ansprüche 1 bis 26 **dadurch gekennzeichnet, dass** das Kriterium die Ableitung der Intensität entlang der Linienrichtung für eine vorbestimmte Anzahl von Linien (16) als bestimmte Bereiche betrifft.

28. Verfahren nach Anspruch 27 **dadurch gekennzeichnet, dass** das Kriterium von einem Pixel (17) erfüllt ist, falls der Absolutwert der Ableitung der Intensität bei diesem Pixel (17) maximal für die Pixel (17) einer Linie (16) ist und einen vorbestimmten Wert überschreitet.

29. Verfahren nach einem der Ansprüche 15 bis 28 **dadurch gekennzeichnet, dass** das Kriterium darin besteht, dass die Farbe der Pixel (17) im Bereich der gelblich-braunen Farbtöne von Brandthefe liegen.

30. Verfahren nach einem der Ansprüche 1 bis 29 **dadurch gekennzeichnet, dass** mehrere festgestellte Maße zum Schluss (27) auf einen Zustand herangezogen werden.

31. Verfahren nach einem der Ansprüche 15 bis 30 **dadurch gekennzeichnet, dass** die Wahrscheinlichkeit für Überweissen gleich 1 minus der Wahrscheinlichkeit für das Ende der Gärung ist.

32. Verfahren nach einem der Ansprüche 1 bis 31 **dadurch gekennzeichnet, dass** auf den Radius des Abbildes der Oberfläche eines Füllmediums (7,8) des Behältnisses (1) und vorzugsweise damit auf die Höhe der Oberfläche innerhalb des Behältnisses (1) geschlossen (27) wird.

33. Verfahren nach Anspruch 32 **dadurch gekennzeichnet, dass** das Kriterium die Ableitung der Intensität entlang radialer vom Abbild der Konusmitte ausgehender Strahlen oder Kreissegmente betrifft.

34. Verfahren nach Anspruch 33 **dadurch gekennzeichnet, dass** das am weitesten au-ßenliegende relative Maximum der Ableitung mit mindestens einem vorgegebenen Absolutwert der Ableitung ein Maß für den Radius des Abbildes der Oberfläche eines Füllmediums (7,8) des Behältnisses (1) entlang eines Strahl darstellt.

35. Verfahren nach Anspruch 34 **dadurch gekennzeichnet, dass** aus der relativen Häufigkeit eines Wertes für den Radius für verschiedene Strahlen auf den Radius für das Abbild der Oberfläche eines Füllmediums (7,8) des Behältnisses (1) geschlossen wird, wobei vorzugsweise darauf geschlossen wird, dass der zu ermittelnde Radius gleich dem Maximum der relativen Häufigkeit eines Wertes für den Radius für verschiedene Strahlen ist.

36. Verfahren nach einem der Ansprüche 1 bis 35 **dadurch gekennzeichnet, dass** der Schluss auf den Zustand in dem Behältnis (1) zur Steuerung bzw. Beendigung des Prozesses in dem Behältnis, zum Befüllen des Behältnisses oder zum Reinigung des Behältnisses verwendet wird.

37. Speichermedium enthaltend ein Computerprogramm oder eine Befehlssequenz die ein Verfahren nach einem der Ansprüche 1 bis 36 realisiert.

38. Rechnergesteuerte Vorrichtung (3,28,29) enthaltend Mittel, die ein Verfahren nach einem der Ansprüche 1 bis 36 ausführen.

## Claims

1. Method for investigating at least one state in a vessel (1), as is used for the production of beverages during the growth and/or fermentation of/by means of organisms, such as for example a fermentation tank, yeast culture tank, biological lactic acid tank, storage tank or the like, in particular a fermentation tank (1) for beer, which comprises the following steps:
- creating (22, 23) at least one image with pixels,
- determining (24) at least one region (16) of the image (15) by selection of pixels (17),
- prescribing (25) at least one criterion, on the basis of which the pixels (17) in the determined region (16) of the image are analysed,
- establishing (26) at least one measure from the position of the pixels (18) and/or from the number of pixels (18) which satisfy the at least one criterion and lie within the determined region (16) and/or the ratio of the number of pixels (18) which satisfy the at least one criterion and lie within the determined region (16) to the number of pixels (17, 18) which lie within the determined region and/or the degree to which the pixels satisfy a criterion, and
- deducing (27) the state in the vessel, taking into account the measure established.

2. Method according to Claim 1, **characterized in that** the vessel (1) has a cylindroconical form with at least one cone (12).

3. Method according to either of Claims 1 and 2, **characterized in that** one criterion concerns the R and/or G and/or B colour value in the RGB colour system.

4. Method according to one of Claims 1 to 3, **characterized in that** one criterion concerns the H and/or S and/or, I colour value in the HSI colour system.

5. Method according to one of Claims 1 to 4, **characterized in that** one criterion concerns the colority and/or the overintensity.

6. Method according to one of Claims 1 to 5, **characterized in that** the image (15) is created by recording.

7. Method according to one of Claims 1 to 6, **characterized in that** the image (15) is created by recording (22) an image (15) and subtracting (23) a reference image from it, in particular pixel by pixel.

8. Method according to either of Claims 6 and 7, **characterized in that** the image (15) is recorded from above in the direction of the lower part (12) of the vessel (1).

9. Method according to one of Claims 1 is 8, **characterized in that** the determined region (16) of the image (15) comprises the depiction of the cone (12) or at least part of the depiction of the cone (12), or consists of the depiction of the cone (12).

10. Method according to one of Claims 1 to 9, **characterized in that** the determined region (16) of the image (15) comprises the depiction of at least one spray head (5) or at least part of the depiction of the spray head (5), or consists of the depiction of at least one spray head (5).

11. Method according to one of Claims 1 to 10, **characterized in that** the determined region (16) of the image comprises the depiction of the region of burnt-on yeast (30) or at least part of the depiction of the region of burnt-on yeast (30), or consists of the depiction of the region of burnt-on yeast (30).

12. Method according to one of Claims 1 to 11, **characterized in that** the determined image region (16) comprises one or more individual sub-image regions.

13. Method according to one of claims 1 to 12, **characterized in that** the determined image region (16) or at least a sub-image region is formed in a circular, linear, square, rectangular, n-cornered or irregular shape.

14. Method according to one of Claims 1 to 13, **characterized in that** the determined image region (16) or at least a sub-image region is obtained by at least omitting an area of the image formed in a circular, linear, square, rectangular, n-cornered or irregular shape.

15. Method according to one of Claims 1 to 14, **characterized in that**
- the state of the inspection glass (6), and/or
- the state of mist in the vessel (1), and/or
- the cleaning state of the vessel (1), and/or
- the filling state, and/or
- the state of the height of the surface of a medium (7, 8) within the vessel (1), and/or
- the state of ventilation, and/or
- the state of submersion of a spray cone (5) into a medium (7, 8) within the vessel (1), and/or
- the state of emptiness of the vessel (1), and/or
- the state of fermentation, such as
- the state of the end of fermentation, and/or
- the state of overwhitening, and/or
- the state of main fermentation
is investigated.

16. Method according to one of Claims 1 to 15, **characterized in that** the deduction (27) of the state in the vessel (1) takes the form of establishing the presence of a state or a probability of the presence of a state.

17. Method according to one of Claims 1 to 16, **characterized in that** the deduction (27) of the state to be investigated is obtained by the measure established going above or below a prescribed value.

18. Method according to either of Claims 16 and 17, **characterized in that** the probability of the presence of a state is equal to the measure established.

19. Method according to one of Claims 1 to 18, **characterized in that** the investigation of a state in the vessel (1) takes place with a single image (15).

20. Method according to one of Claims 1 to 19, **characterized in that** the investigation of the state in the vessel (1) takes place with a number of successive images (15), and in particular also their time sequence or interval.

21. Method according to one of Claims 1 to 20, **characterized in that** the coordinates and/or the radius of the cone (12) and/or of a spray ball (5) are prescribed.

22. Method according to one of Claims 1 to 21, **characterized in that** the contrast and the brightness with which the image (15) is recorded are prescribed.

23. Method according to one of Claims 14 to 22, **characterized in that** the state of the inspection glass (6) is investigated by determining an image region (16) in which a great difference in intensity occurs, preferably in the region of the depiction of a spray head (5) or a feed line (4) to the spray head (5), when the inspection glass (6) is clean and transparent.

24. Method according to Claim 23, **characterized in that** the criterion concerns the difference in intensity between the intensities of the pixels (17) of the lines (16) of a pair of lines for a predetermined number of pairs of lines.

25. Method according to Claim 24, **characterized in that**, if the degree of the difference in intensity for a pixel (17) goes above or below a predetermined value and no other pixel (17) of the same line (16) goes above or below the predetermined value, the criterion is satisfied by such a pixel (17).

26. Method according to one of Claims 1 to 25, **characterized in that**, for the creation (23) of an image (15), a sharpening operation is carried out on a recorded image (15).

27. Method according to one of Claims 1 to 26, **characterized in that** the criterion concerns the derivative of the intensity along the direction of the line for a predetermined number of lines (16) as determined regions.

28. Method according to Claim 27, **characterized in that** the criterion is satisfied by a pixel (17) if the absolute value of the derivative of the intensity in the case of this pixel (17) is at a maximum for the pixels (17) of a line (16) and goes above a predetermined value.

29. Method according to one of Claims 15 to 28, **characterized in that** the criterion consists **in that** the colour of the pixels (17) lies in the range of the yellowish-brown colour tones of burnt-on yeast.

30. Method according to one of Claims 1 to 29, **characterized in that** a number of established measures are used for the deduction (27) of a state.

31. Method according to one of Claims 15 to 30, **characterized in that** the probability of overwhitening is equal to 1 minus the probability of the end of the fermentation.

32. Method according to one of Claims 1 to 31, **characterized in that** the radius of the depiction of the surface of a filling medium (7, 8) of the vessel (1) is deduced (27), and preferably with it the height of the surface within the vessel (1).

33. Method according to Claim 32, **characterized in that** the criterion concerns the derivative of the intensity along radial half lines or segments of a circle extending from the depiction of the centre of the cone.

34. Method according to Claim 33, **characterized in that** the furthest outlying relative maximum of the derivative with at least one prescribed absolute value of the derivative represents a measure of the radius of the depiction of the surface of a filling medium (7, 8) of the vessel (1) along a half line.

35. Method according to Claim 34, **characterized in that** the radius for the depiction of the surface of a filling medium (7, 8) of the vessel (1) is deduced from the relative frequency of a value for the radius for various half lines, it preferably being deduced that the radius to be determined is equal to the maximum of the relative frequency of a value for the radius for various half lines.

36. Method according to one of Claims 1 to 35, **characterized in that** the deduction of the state in the vessel (1) is used for controlling or ending the process in the vessel, for filling the vessel or for cleaning the vessel.

37. Storage medium containing a computer program or a command sequence which realizes a method according to one of Claims 1 to 36.

38. Computer-controlled device (3, 28, 29) containing means which perform a method according to one of Claims 1 to 36.

## Revendications

1. Procédé d'analyse d'au moins un état dans un récipient (1), tel qu'il est utilisé dans la fabrication de boissons lors du développement d'organismes et/ou de la fermentation avec des organismes, comme par exemple une cuve de fermentation, un bac de culture de levure biologique pure, un réservoir d'acide lactique biologique, un réservoir de stockage ou autres, en particulier une cuve de fermentation (1) pour de la bière, comprenant les phases suivantes :
- élaboration (22, 23) d'au moins une image composée de pixels,
- définition (24) d'au moins une zone (16) de l'image (15) par sélection de pixels (17),
- prédéfinition (25) d'au moins un critère, à l'aide duquel les pixels (17) sont analysés dans la zone définie (16) de l'image,
- établissement (26) d'au moins une mesure à partir de la position des pixels (18) et/ou du nombre de pixels (18), qui remplissent le au moins critère et se situent à l'intérieur de la zone définie (16) et/ou du rapport entre le nombre de pixels (18), qui remplissent le au moins critère et se situent à l'intérieur de la zone définie (16), et le nombre de pixels (17, 18), qui se situent à l'intérieur de la zone définie, et/ou de l'intensité avec laquelle des pixels remplissent un critère, et
- conclusion (27) sur l'état dans le récipient compte tenu de la mesure établie.

2. Procédé suivant la revendication 1, **caractérisé en ce que** le récipient (1) a une forme cylindro-conique avec au moins un cône (12).

3. Procédé suivant l'une des revendications 1 et 2, **caractérisé en ce qu'**un critère concerne la valeur chromatique R et/ou V et/ou B dans le système RVB (rouge - vert - bleu).

4. Procédé suivant l'une des revendications 1 à 3, **caractérisé en ce qu'**un critère concerne la valeur chromatique H et/ou S et/ou I dans le système HSI (Hue, Saturation, Intensity).

5. Procédé suivant l'une des revendications 1 à 4, **caractérisé en ce qu'**un critère concerne la coloration et/ou la saturation.

6. Procédé suivant l'une des revendications 1 à 5, **caractérisé en ce que** l'image (15) est élaborée par prise de vue.

7. Procédé suivant l'une des revendications 1 à 6, **caractérisé en ce que** l'image (15) est élaborée du fait qu'une image (15) est filmée (22) et une image de référence est soustraite (23) de cette dernière en particulier par pixels.

8. Procédé suivant l'une des revendications 6 et 7, **caractérisé en ce que** l'image (15) est filmée à partir du haut en direction de la partie inférieure (12) du récipient (1).

9. Procédé suivant l'une des revendications 1 à 8, **caractérisé en ce que** la zone définie (16) de l'image (15) comprend la reproduction du cône (12) ou au moins une partie de la reproduction du cône (12) ou est constituée de la reproduction du cône (12).

10. Procédé suivant l'une des revendications 1 à 9, **caractérisé en ce que** la zone définie (16) de l'image (15) comprend la reproduction d'au moins une tête de pulvérisation (5) ou au moins une partie de la reproduction de la tête de pulvérisation (5) ou est constituée de la reproduction d'au moins une tête de pulvérisation (5).

11. Procédé suivant l'une des revendications 1 à 10, **caractérisé en ce que** la zone définie (16) de l'image comprend la reproduction de la zone de levure brûlée (30) ou au moins une partie de la reproduction de la zone de levure brûlée (30) ou est constituée de la reproduction de la zone de levure brûlée (30).

12. Procédé suivant l'une des revendications 1 à 11, **caractérisé en ce que** la zone d'image définie (16) comprend une ou plusieurs zones d'images partielles individuelles.

13. Procédé suivant l'une des revendications 1 à 12, **caractérisé en ce que** la zone d'image définie (16) ou au moins une zone d'images partielles est de forme circulaire, linéaire, carrée, rectangulaire, multi-angulaire ou irrégulière.

14. Procédé suivant l'une des revendications 1 à 13, **caractérisé en ce que** la zone d'image définie (16) ou au moins une zone d'images partielles est obtenue par au moins omission d'une région d'image de forme circulaire, linéaire, carrée, rectangulaire, multi-angulaire ou irrégulière.

15. Procédé suivant l'une des revendications 1 à 14, **caractérisé par** l'analyse
- de l'état du verre de regard (6) et/ou
- de l'état du brouillard dans le récipient (1), et/ou
- de l'état de nettoyage dans le récipient (1), et/ou
- de l'état du remplissage, et/ou
- de l'état du niveau de la surface d'un fluide (7, 8) à l'intérieur du récipient (1), et/ou
- de l'état de l'aération, et/ou
- de l'état d'immersion d'une bille de pulvérisation (5) dans un fluide (7, 8) à l'intérieur du récipient (1), et/ou
- de l'état de vide du récipient (1), et/ou
- de l'état de la fermentation, tel que
- l'état de la fin de la fermentation, et/ou
- l'état du surblanchiment, et/ou
- l'état de la fermentation principale.

16. Procédé suivant l'une des revendications 1 à 15, **caractérisé en ce que** la conclusion (27) sur l'état dans le récipient (1) s'effectue en forme d'un constat de la présence d'un état ou d'une probabilité pour la présence d'un état.

17. Procédé suivant l'une des revendications 1 à 16, **caractérisé en que** le dépassement par le haut ou par le bas de la mesure établie au-dessus ou au-dessous d'une valeur prédéfinie fournit la conclusion (27) sur l'état à analyser.

18. Procédé suivant l'une des revendications 16 et 17, **caractérisé en ce que** la probabilité pour la présence d'un état est égale à la mesure établie.

19. Procédé suivant l'une des revendications 1 à 18, **caractérisé en ce que** l'analyse d'un état dans le récipient (1) s'effectue avec une image individuelle (15).

20. Procédé suivant l'une des revendications 1 à 19, **caractérisé en ce que** l'analyse de l'état dans le récipient (1) s'effectue avec plusieurs images successives (15) et en particulier également avec leur succession ou écart dans le temps.

21. Procédé suivant l'une des revendications 1 à 20, **caractérisé en ce que** les coordonnées et/ou le rayon du cône (12) et/ou d'une bille de pulvérisation (5) sont prédéfinis.

22. Procédé suivant l'une des revendications 1 à 21, **caractérisé en ce que** le contraste et la luminosité, avec lesquels est filmée l'image (15), sont prédéfinis.

23. Procédé suivant l'une des revendications 14 à 22, **caractérisé en ce que** l'état du verre de regard (6) est analysé par définition d'une zone d'image (16), une différence d'intensité élevée apparaissant de préférence dans la zone de la reproduction d'une tête de pulvérisation (5) ou d'une conduite d'arrivée (4) à la tête de pulvérisation (5) en présence d'un verre de regard (6) propre et transparent.

24. Procédé suivant la revendication 23, **caractérisé en ce que** le critère concerne la différence d'intensités entre les intensités des pixels (17) des lignes (16) d'une paire de lignes pour un nombre prédéfini de paires de lignes.

25. Procédé suivant la revendication 24, **caractérisé en ce que**, si l'intensité de la différence d'intensité pour un pixel (17) dépasse par le haut ou par le bas une valeur prédéfinie et si aucun autre pixel (17) de la même ligne (16) dépasse par le haut ou par le bas la valeur prédéfinie, le critère est rempli par un tel pixel (17).

26. Procédé suivant l'une des revendications 1 à 25, **caractérisé en ce qu'**une opération de mise au point d'une image filmée (15) est effectuée pour l'élaboration (23) d'une image (15).

27. Procédé suivant l'une des revendications 1 à 26, **caractérisé en ce que** le critère concerne la dérivée de l'intensité le long de la direction des lignes pour un nombre prédéfini de lignes (16) en tant que zones définies.

28. Procédé suivant la revendication 27, **caractérisé en ce que** le critère est rempli par un pixel (17), si la valeur absolue de la dérivée de l'intensité pour ce pixel (17) est maximale pour les pixels (17) d'une ligne (16) et dépasse par le haut une valeur prédéfinie.

29. Procédé suivant l'une des revendications 15 à 28, **caractérisé en ce que** le critère consiste **en ce que** la couleur des pixels (17) se situe dans la gamme des teintes jaune - brun de levure brûlée.

30. Procédé suivant l'une des revendications 1 à 29, **caractérisé en ce que** plusieurs mesures établies sont utilisées pour la conclusion (27) sur un état.

31. Procédé suivant l'une des revendications 15 à 30, **caractérisé en ce que** la probabilité de surblanchiment est égale à 1 moins la probabilité pour la fin de la fermentation.

32. Procédé suivant l'une des revendications 1 à 31, **caractérisé en ce qu'**il est conclu (27) du rayon de la reproduction de la surface d'un fluide de remplissage (7, 8) du récipient (1) et de préférence ainsi du niveau de la surface à l'intérieur du récipient (1).

33. Procédé suivant la revendication 32, **caractérisé en ce que** le critère concerne la dérivée de l'intensité le long de faisceaux radiaux partant de la reproduction du centre du cône ou de segments de cercle.

34. Procédé suivant la revendication 33, **caractérisé en ce que** le maximum relatif le plus extérieur de la dérivée représente, avec au moins une valeur absolue prédéfinie de la dérivée, une mesure pour le rayon de la reproduction de la surface d'un fluide de remplissage (7, 8) du récipient (1) le long d'un faisceau.

35. Procédé suivant la revendication 34, **caractérisé en ce qu'**il est conclu du rayon pour la reproduction de la surface d'un fluide de remplissage (7, 8) du récipient (1) à partir de la fréquence relative d'une valeur pour le rayon pour différents faisceaux, il étant alors de préférence conclu que le rayon à déterminer est égal au maximum de la fréquence relative d'une valeur pour le rayon pour différents faisceaux.

36. Procédé suivant l'une des revendications 1 à 35, **caractérisé en ce que** la conclusion de l'état dans le récipient (1) est utilisée pour la commande ou l'achèvement du processus dans le récipient, pour le remplissage ou pour le nettoyage de ce dernier.

37. Support d'information comprenant un programme d'ordinateur ou une séquence d'instructions qui réalise un procédé suivant l'une des revendications 1 à 36.

38. Dispositif (3, 28, 29) piloté par ordinateur, comprenant des moyens qui réalisent un procédé suivant l'une des revendications 1 à 36.
